(19) Europäisches Patentamt
European Patent Office
Office européen des brevets

(11) EP 0 616 518 B1

(12) **EUROPÄISCHE PATENTSCHRIFT**

(45) Veröffentlichungstag und Bekanntmachung des Hinweises auf die Patenterteilung:
**17.06.1998 Patentblatt 1998/25**

(21) Anmeldenummer: 93919279.5

(22) Anmeldetag: **06.09.1993**

(51) Int. Cl.⁶: **A61K 7/06**, A61K 7/13

(86) Internationale Anmeldenummer:
**PCT/EP93/02406**

(87) Internationale Veröffentlichungsnummer:
**WO 94/08554 (28.04.1994 Gazette 1994/10)**

(54) **MITTEL ZUR FESTIGUNG DER HAARE**

HAIR-FIXING AGENT

AGENT DE FIXATION CAPILLAIRE

(84) Benannte Vertragsstaaten:
**DE ES FR GB IT**

(30) Priorität: **15.10.1992 DE 4234743**

(43) Veröffentlichungstag der Anmeldung:
**28.09.1994 Patentblatt 1994/39**

(73) Patentinhaber:
**Wella Aktiengesellschaft**
**64274 Darmstadt (DE)**

(72) Erfinder:
- **LANG, Günther, Dr.**
  **D-64354 Reinheim (DE)**
- **CLAUSEN, Thomas, Dr.**
  **D-64665 Alsbach (DE)**

(56) Entgegenhaltungen:
| | |
|---|---|
| **EP-A- 0 242 792** | **EP-A- 0 272 472** |
| **EP-A- 0 288 012** | **EP-A- 0 412 744** |
| **WO-A-91/15186** | **WO-A-92/21319** |
| **DE-A- 1 467 925** | **DE-A- 2 003 487** |
| **GB-A- 2 206 045** | **US-A- 3 959 463** |

**Beschreibung**

Die vorliegende Erfindung betrifft ein Mittel zur Festigung der Haare auf der Basis einer wasserhaltigen Lösung mindestens eines filmbildenden natürlichen Polymers, welches eine Kombination aus einem wasserlöslichen, halogenfreien organischen Lösungsmittel und mindestens einer bestimmten bei Raumtemperatur wasserunlöslichen, im Mittel zur Festigung der Haare jedoch löslichen Verbindung enthält.

An Mittel zur Festigung der Haare sind eine Vielzahl von Anforderungen zu stellen. So müssen diese Mittel dem Haar einen festen und flexiblen Halt geben ohne den natürlichen Griff des Haares zu beeinträchtigen. Auch bei hoher Luftfeuchtigkeit müssen die Festigungseigenschaften des Mittels erhalten bleiben, damit sich das Haar nicht klebrig anfühlt. Gleichzeitig muß jedoch auch gewährleistet sein, daß auch durch wiederholtes Bleichen, Dauerwellen, Färben oder durch häufiges Waschen mit entfettenden Haarreinigungsmitteln strapaziertes Haar sich nach dem Auftragen des Mittels zur Festigung der Haare, insbesondere im nassen Haarzustand, leicht kämmen läßt. Das zuvor mit dem Mittel zur Festigung behandelte, getrocknete Haar sollte, neben einem guten Halt der Frisur, einen schönen Glanz aufweisen.

Bisher konnten den üblicherweise in Form einer wäßrigen oder wäßrig-alkoholischen Lösung vorliegenden Mitteln zur Festigung der Haare nur unzureichende Mengen kämmbarkeitsverbessernd wirkender Pflegestoffe zugesetzt werden, ohne die Festigungseigenschaften und die Homogenität des Mittels nachteilig zu beeinflussen.

In der Vergangenheit wurden daher auch zweiphasige Mittel zur Festigung der Haare, sogenannte Schüttelemulsionen, zur Verfügung gestellt. Bei diesen Schüttelemulsionen wurde die wäßrige oder wäßrigalkoholische Festigerphase mit einer zweiten, Pflegestoffe enthaltenden, wasserunlöslichen Ölphase, beispielsweise aus Paraffinöl, überschichtet. Durch Schütteln wurde vor der Anwendung dieser Mittel eine instabile Emulsion erzeugt. Derartige Schüttelemulsionen haben jedoch den Nachteil, daß es aufgrund der Instabilität der gebildeten Emulsion zu einer ungleichmäßigen Verteilung von festigenden und pflegenden Bestandteilen auf dem Haar kommt. Die als Schüttelemulsionen vorliegenden Mittel zur Festigung der Haare konnten daher hinsichtlich der Festigung und der gleichzeitigen Verbesserung der Naßkämmbarkeit nicht befriedigen. Zudem sind derartige Schüttelemulsionen nur in Einportionspackungen im Handel, was im Hinblick auf ökologische Aspekte und die Abfallvermeidung nicht sinnvoll erscheint.

Aus der WO 91/15186 ist ein Haarbehandlungsmittel bekannt, welches gleichzeitig haarkonditionierende und haarfestigende Eigenschaften aufweist. Hierfür werden synthetische Polymere mit einem haarkonditionierenden Wirkstoff kombiniert, welcher unter anderem ein Lipid, ein kationisches Tensid, ein Proteinderivat oder ein Siloxan sein kann.

Aus der EP-O 242 792 A2 ist ein haarkosmetisches Mittel bekannt, welches dem Haar gute Flexibilität, Weichheit und antistatische Aufladung verleiht. Hierfür wird ein näher bezeichnetes, verzweigtes, quaternäres Ammoniumsalz mit einer näher bezeichneten Ölkomponente kombiniert. In Beispiel 8 ist das haarkosmetische Mittel durch einen zusätzlichen Gehalt an einem Methacrylesterpolymer als Haarfestigungsmittel formuliert.

In der DE-OS 1 467 925 ist ein Frisierhilfsmittel beschrieben, welches dem Haar eine gute Elastizität verleiht. Dies wird durch eine Kombination eines Vinylacetat/Glykol-Copolymers mit Panthenol oder Panthenolether erreicht. Als optionaler Zusatzbestandteil können bestimmte $C_{11}$-, $C_{15}$- und $C_{20}$-Triole enthalten sein.

Aus der EP-A-0 272 472 ist ein Haarfestigungsmittel bekannt, welches Chitosan und ein ampholytisches Copolymerisat enthält.

Es bestand daher die Aufgabe, ein Mittel zur Festigung der Haare auf der Basis einer wasserhaltigen Lösung zur Verfügung zu stellen, das die Nachteile der bisher bekannten Mittel nicht aufweist, das heißt sowohl eine gute Festigung der Haare als auch eine gute Kämmbarkeit, insbesondere Naßkämmbarkeit, des Haares bewirkt.

Überraschenderweise wurde nun gefunden, daß durch den Zusatz einer Kombination aus mindestens einem wasserlöslichen, halogenfreien organischen Lösungsmittel und einer bei Raumtemperatur wasserunlöslichen, im Mittel jedoch löslichen Verbindung zu einer wasserhaltigen, mindestens ein filmbildendes Polymer enthaltenden Lösung, ein Mittel zur Festigung der Haare erhalten wird, das alle an ein derartiges Mittel gestellten Anforderungen in hervorragender Weise erfüllt.

Gegenstand der vorliegenden Erfindung ist daher ein Mittel zur Festigung der Haare auf der Basis einer wasserhaltigen Lösung mindestens eines fimbildenden natürlichen Polymers, wobei das filmbildende Polymer ausgewählt ist aus Chitinderivaten, Chitosan, Chitosanderivaten, Chitosansalzen, Schellack, Alginaten, Gelatine und Pektinen und wobei das Mittel zusätzlich einen Gehalt an einer Kombination aus

(A) mindestens einem wasserlöslichen, halogenfreien organischen Lösungsmittel und
(B) 0,05 bis 2 Gewichtsprozent mindestens einer bei Raumtemperatur wasserunlöslichen, im Mittel jedoch löslichen Verbindung, welche ausgewählt ist aus $C_8$- bis $C_{20}$-Alkandiolen, $C_8$- bis $C_{20}$-Alkantriolen, den Mono- oder Monodiglyceriden der gesättigten oder ungesättigten $C_{12}$- bis $C_{20}$-Fettsäuren, den mit 2 oder 3 Mol Ethylenoxid ethoxylierten $C_{12}$- bis $C_{20}$-Fettalkoholen, Polysiloxan-Polyether-Copolymeren mit einem Trübungspunkt bei einer Temperatur unter 20° Celsius, Polyoxyethylen-Polyoxypropylen-Blockpolymeren oder Polyoxyethylen-Polyoxybutylen-Blockpolymeren,

wobei das Gewichtsverhältnis der Komponente (A) zur Komponente (B) 1000:1 bis 2,5:1, vorzugsweise 100:1 bis 2,5:1 beträgt, aufweist.

Unter Raumtemperatur wird in der vorliegenden Anmeldung eine Temperatur von 20 bis 25° Celsius verstanden.

Das Mittel zur Festigung der Haare gemäß der vorliegenden Erfindung bewirkt bei guter Festigung gleichzeitig eine überraschende und ausgezeichnete Verbesserung sowohl der Kämmbarkeit, insbesondere des feuchten Haares, als auch von Glanz und Griff des trockenen Haares.

Insbesondere wird die Gleitwirkung von Kamm oder Bürste im nassen Haar nach der Applikation des erfindungsgemäßen Mittels wesentlich verbessert, wodurch das Fönen der Haare oder das Aufwickeln auf Wasserwellwickler wesentlich erleichtert wird.

Das wasserlösliche, halogenfreie organische Lösungsmittel der Komponente (A) ist bevorzugt ausgewählt aus $C_1$- bis $C_4$-Alkoholen, wie Ethanol, Propanol, Isopropanol, $C_1$- bis $C_4$-Glykolen, wie Ethylenglykol oder Propylenglykol, Polyethylenglykolestern mit $C_2$- bis $C_{20}$-Carbonsäuren, flüssigen Polyethylenglykolen, Polyethylenglykolalkylethern von $C_1$- bis $C_4$-Alkoholen, einfachen oder gemischten Ketonen von $C_1$- bis $C_5$-Alkoholen, wie beispielsweise Aceton oder Methylethylketon, wobei aliphatische $C_1$- bis $C_4$-Alkohole, $C_1$- bis $C_4$-Glykole, Polyethylglykolester mit $C_2$- bis $C_{20}$-Carbonsäuren, flüssige Polyethylenglykole oder deren Gemische besonders bevorzugt sind.

Das erfindungsgemäße Mittel enthält bevorzugt 5 bis 60 Gewichtsprozent, besonders bevorzugt 10 bis 50 Gewichtsprozent, des wasserlöslichen, halogenfreien organischen Lösungsmittels der Komponente (A).

Als wasserunlösliches $C_8$- bis $C_{20}$-Alkandiol oder wasserunlösliches $C_8$- bis $C_{20}$-Alkantriol der Komponente (B) enthält das erfindungsgemäße Mittel bevorzugt wasserunlösliche gerad- oder verzweigtkettige, gesättigte oder ungesättigte 1,2-$C_8$- bis $C_{20}$-Alkandiole oder 1,2,3-$C_8$- bis $C_{20}$-Alkantriole, wie beispielsweise 1,2-Octandiol, 1,2-Dodecandiol, 1,2-Hexadecandiol oder 1,2,3-Trihydroxy-3,7,11,15-tetramethylhexadecan. Von den als Komponente (B) geeigneten wasserunlöslichen Alkandiolen und Alkantriolen ist das 1,2,3-Trihydroxy-3,7,11,15-tetramethylhexadecan, das beispielsweise in Form des Handelsproduktes Phytantriol® von der Firma Hoffmann La-Roche, Basel/Schweiz vertrieben wird, besonders bevorzugt.

Von den Mono- oder Monodiglyceriden der gesättigten $C_{12}$- bis $C_{20}$-Fettsäuren stellen bevorzugt Glycerinmonolaurat, Glycerinmonooleat oder Palmkernölmonoglycerid die Komponente (B) des erfindungsgemäßen Mittels dar.

Als Glycerinmonolaurat oder -monooleat sollen bevorzugt Handelsprodukte mit einem Monoglyceridanteil von 80 Prozent eingesetzt werden, während als Palmkernölmonoglycerid vorzugsweise Handelsprodukte mit einem Monoglyceridanteil von 60 Prozent, als Komponente (B) geeignet sind.

Der mit 2 oder 3 Mol Ethylenoxid ethoxylierte $C_{12}$- bis $C_{20}$-Fettalkohol der Komponente (B) des erfindungsgemäßen Mittels ist bevorzugt mit 2 oder 3 Mol Ethylenoxid ethoxylierter Lauryl-, Myristyl-, Cetyl- oder Stearylalkohol oder deren Gemisch, besonders bevorzugt jedoch mit 2 oder 3 Mol Ethylenoxid ethoxylierter Laurylalkohol.

Als Polysiloxan-Polyether-Copolymere mit einem Trübungspunkt bei einer Temperatur unter 20° Celsius (gemessen in 4 prozentiger wäßriger Lösung) der Komponente (B) des erfindungsgemäßen Mittels ist bevorzugt ein Dimethylpolysiloxan-Polyethylenoxid-Polypropylenoxid-Copolymer mit einem Trübungspunkt von 10° Celsius, das beispielsweise von der Firma Th. Goldschmidt AG, Essen/BRD unter der Handelsbezeichnung Abil B 8852® vertrieben wird, geeignet.

Von den Polyoxyethylen-Polyoxypropylen-Blockpolymere, die als Komponente (B) in dem erfindungsgemäßen Mittel enthalten sein können, sind jene bevorzugt, die einen Polyoxyethylenanteil am Gesamtmolekulargewicht von 10 Prozent aufweisen und beispielsweise von der Firma BASF, Ludwigshafen/BRD unter den Handelsbezeichnungen Pluronic L 61®, Pluronic L 81®, Pluronic L 101® und Pluronic L 121® vertrieben werden.

In einer bevorzugten Ausführungsform des erfindungsgemäßen Mittels ist die bei Raumtemperatur wasserunlösliche, im Mittel jedoch lösliche Verbindung der Komponente (B) ausgewählt aus 1,2 Octandiol, 1,2-Dodecandiol, 1,2-Hexadecandiol, 1,2,3-Trihydroxy-3,7,11,15-tetramethylhexadecan, Glycerinmonolaurat, Glycerinmonooleat, Palmkernölmonoglycerid, mit 2 oder 3 Molen Ethylenoxid ethoxyliertem Laurylalkohol, Dimethylpolysiloxan-Polyethylenoxid-Polypropylenoxid-Copolymeren mit einem Trübungspunkt bei 10° Celsius und Polyoxyethylen-Polyoxypropylen-Blockpolymeren mit einem Polyethylenoxidanteil am Gesamtmolekulargewicht von 10 Prozent.

Das erfindungsgemäße Mittel enthält bevorzugt 0,1 bis 0,5 Gewichtsprozent der Komponente (B).

Das erfindungsgemäße Mittel enthält bevorzugt mindestens ein natürliches Polymer, ausgewählt aus Chitinderivaten, Chitosan, Chitosandervaten und Chitosansalzen allein oder im Gemisch mit mindestens einem synthetischen filmbildenden Polymer, beispielsweise anionische Polymere wie Acrylsäure- oder Methacrylsäure-Homopolymere oder -Copolymere, Copolymerisate aus Acrylsäure und Acrylamiden und Copolymere auf der Basis von Alkylvinylethern und Maleinsäuremonoalkylestern; amphotere Polymere wie Copolymerisate aus Octylacrylamid, Acrylat und Butylaminoethylmethacrylat; kationische Polymere wie Vinylimidazolinium-Vinylpyrrolidon-Copolymere; nichtionische Polymere wie Vinylpyrrolidon-Homopolymere, Vinylpyrrolidon-Vinylacetat-Copolymere oder Terpolymere aus Vinylpyrrolidon, Vinylacetat und Vinylpropionat.

Die in dem erfindungsgemäßen Mittel enthaltenden filmbildenden Polymere können, sofern diese Polymere saure Gruppen enthalten, in einer zu 50 bis 100 Prozent mit organischen Aminen oder Alkanolaminen, bevorzugt mit 2-Amino-2-methyl-1-propanol, 2-Amino-1-butanol oder Triisopropylamin, neutralisierten Form eingesetzt werden.

Das erfindungsgemäße Mittel enthält das filmbildende natürliche oder synthetische Polymer vorzugsweise in einer Menge von 0,1 bis 5 Gewichtsprozent, besonders bevorzugt 0,5 bis 2,5 Gewichtsprozent.

Das erfindungsgemäße Mittel zur Festigung der Haare kann gegebenenfalls durch einen Gehalt an direkt auf das Haar aufziehenden Farbstoffe das Haar gleichzeitig färben oder tönen. Derartige Präparate sind unter anderem als Farbfestiger oder Tönungsfestiger im Handel bekannt. Sie enthalten zusätzlich übliche direkt auf das Haar aufziehende Farbstoffe, beispielsweise aromatische Nitrofarbstoffe, zum Beispiel 1,4-Diamino-2-nitro-benzol, Pikraminsäure, 1-Hydroxy-2-amino-4-nitrobenzol und 1,4-bis(2-Hydroxyethyl)amino-2-nitro-5-chlor-benzol, Azofarbstoffe, zum Beispiel Acid Brown 4 (C.I.14 805), Anthrachinonfarbstoffe, zum Beispiel Disperse Violet 4 (C.I. 61 105) und Triphenylmethanfarbstoffe, zum Beispiel Basic Violet 1 (C.I. 42 535), Basic Violet 14 (C.I. 42 510) oder Basic Blue 7 (C.I. 42 595:1), wobei die Farbstoffe dieser Klassen, je nach Art ihrer Substituenten, sauren, nichtionogenen oder basischen Charakter haben können. Ihre Gesamtkonzentration in dem erfindungsgemäßen Mittel beträgt üblicherweise etwa 0,01 bis 2,0 Gewichtsprozent.

Das erfindungsgemäße Mittel liegt in Form einer wasserhaltigen Zubereitung, insbesondere als Lösung, Schaum oder Gel, vor, wobei der Wassergehalt des Mittels bevorzugt 30 bis 95 Gewichtsprozent beträgt.

Das erfindungsgemäße Mittel zur Festigung der Haare kann in Form eines mit Hilfe einer geeigneten mechanisch betriebenen Sprühvorrichtung versprühbaren Non-Aerosol-Schaumes vorliegen.

Unter mechanischen Sprühvorrichtungen sind solche Vorrichtungen zu verstehen, welche das Versprühen einer Flüssigkeit ohne Verwendung eines verflüssigten Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem sich das Mittel infolge der Kontraktion des elastischen Behälters bei Öffnen des Sprühventils kontinuierlich entnehmen läßt, verwendet werden.

Das erfindungsgemäße Mittel kann auch unter Zusatz eines Treibmittels in einem Druckbehälter abgefüllt werden, wobei das Präparat als Schaum entnommen wird und mittels eines mit einer Auftragsdüse versehenen Ventils leicht dosiert und bequem auf dem Haar verteilt werden kann. Als Treibmittel sind beispielsweise leicht flüchtige Fluorchlorkohlenwasserstoffe, wie zum Beispiel Difluordichlormethan oder Trichlormonofluormethan, Tetrafluordichlorethan oder niedere Alkane, wie zum Beispiel n-Butan, i-Butan und Propan oder auch Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Verbindungen geeignet. Die Treibmittel sind in diesen Mittel zweckmäßigerweise in einer Menge von etwa 2 bis 10 Gewichtsprozent enthalten.

Weiterhin kann das erfindungsgemäße Mittel für Haarfestigungsmittel übliche kosmetische Zusatzstoffe enthalten, wie anionische, kationische, amphotere oder nicht-ionische Netzmittel und Emulgatoren, wie zum Beispiel $C_{12}$- bis $C_{18}$-Alkylethersulfate, Alkyltrimethylammoniumsalze, Alkylpyridiniumsalze, Carboxyderivate des Imidazols, N-Alkylsulfobetaine, Polyglycerylether von gesättigten oder ungesättigten Fettalkoholen und Alkylphenole, in einer Menge von etwa 0,01 bis 3 Gewichtsprozent; ferner Konservierungsstoffe, wie zum Beispiel Salicylsäure oder Mandelsäure, in einer Menge von 0,01 bis 0,7 Gewichtsprozent; sowie Antischuppenwirkstoffe, wie zum Beispiel Zink-Pyridinthion; weiterhin haarpflegende Wirkstoffe, wie zum Beispiel Fettsäureester, Fettalkohole, Lanolinderivate oder Pantothensäure, in einer Menge von etwa 0,01 bis 3 Gewichtsprozent; hydrophobierende Substanzen wie Siliconöle, beispielsweise Polydimethylsiloxane, Polymethylphenylsiloxane oder Cyclomethicon; Weichmacher wie Phthalsäureester oder Alkylcitrate; kationische Polymere, zum Beispiel kationische Cellulosederivate, in einer Menge von 0,01 bis 0,2 Gewichtsprozent sowie ferner Komplexbildner, Schaumstabilisatoren, Puffersubstanzen, Lichtschutzmittel oder Parfümöle in einer Menge von etwa 0,01 bis 0,8 Gewichtsprozent.

Das erfindungsgemäße Mittel enthält bevorzugt 0,01 bis 2 Gewichtsprozent mindestens eines für Haarfestigungsmittel üblichen kosmetischen Zusatzstoffs.

Das erfindungsgemäße Mittel wird, üblicherweise nach der Haarwäsche, in dem handtuchtrockenen Haar je nach Haarfülle in einer Menge von etwa 5 bis 20 g verteilt. Anschließend wird das Haar durchgekämmt und in üblicherweise entweder unmittelbar gefönt oder zunächst auf Wasserwellwickler gewickelt und sodann getrocknet.

Die nachstehenden Beispiele sollen den Gegenstand der Erfindung näher erläutern.

**Beispiele**

**Beispiel 1: Haarfestiger**

| | |
|---|---|
| 0,60 g | Chitosan |
| 0,50 g | mit 3 Mol Ethylenoxid ethoxylierter Oleylalkohol |
| 1,52 g | Ameisensäure |
| 0,75 g | Parfümöl |

| | |
|---|---|
| 16,00 g | Isopropanol |
| 80,63 g | Wasser |
| 100,00 g | |

**Beispiel 2: Haarfestiger**

| | |
|---|---|
| 0,60 g | Chitosan |
| 0,50 g | mit 2 Mol Ethylenoxid ethoxylierter Oleylalkohol |
| 1,52 g | Ameisensäure |
| 0,75 g | Parfümöl |
| 24,00 g | Isopropanol |
| 72,63 g | Wasser |
| 100,00 g | |

**Beispiel 3: Haarfestiger**

| | |
|---|---|
| 0,60 g | Chitosan |
| 0,50 g | mit 3 Mol Ethylenoxid ethoxylierter Laurylalkohol |
| 1,52 g | Ameisensäure |
| 0,75 g | Parfümöl |
| 12,00 g | Isopropanol |
| 84,63 g | Wasser |
| 100,00 g | |

**Beispiel 4: Haarfestiger**

| | |
|---|---|
| 2,00 g | Polyvinylpyrrolidon |
| 0,20 g | Chitosan |
| 0,45 g | Ölsäuremonoglycerid |
| 0,90 g | Parfümöl |
| 28,00 g | Isopropanol |
| 68,45 g | Wasser |
| 100,00 g | |

**Beispiel 5: Haarfestiger**

| | |
|---|---|
| 2,00 g | Polyvinylpyrrolidon |
| 0,20 g | Chitosan |
| 0,50 g | Dimethylpolysiloxan-Polyethylenoxid-Polypropylenoxid-Copolymer (zum Beispiel Abil B 8852® der Firma Th. Goldschmidt/BRD) |
| 0,90 g | Parfümöl |
| 33,00 g | Isopropanol |
| 63,40 g | Wasser |
| 100,00 g | |

**Beispiel 7: Haarfestigungsschaum**

| | |
|---|---|
| 2,800 g | Chitosan |
| 0,190 g | Glycerinmonolaurat |
| 0,200 g | Cetyltrimethylammoniumchlorid |
| 0,688 g | Ameisensäure |
| 0,200 g | Parfümöl |
| 22,000 g | Ethanol |
| 73,922 g | Wasser |
| 100,000 g | |

**Beispiel 8: Haarfestigungsschaum**

| | |
|---|---|
| 2,800 g | Chitosan |
| 0,200 g | 1,2-Lauryldiol |
| 0,200 g | Cetyltrimethylammoniumchlorid |
| 0,688 g | Ameisensäure |
| 0,200 g | Parfümöl |
| 22,000 g | Ethanol |
| 73,912 g | Wasser |
| 100,000 g | |

**Beispiel 9: Haarfestigungsschaum**

| | |
|---|---|
| 2,800 g | Chitosan |
| 0,200 g | mit 2 Mol Ethylenoxid ethoxylierter Cetylalkohol |
| 0,200 g | Cetyltrimethylammoniumchlorid |
| 0,688 g | Ameisensäure |
| 0,200 g | Parfümöl |
| 22,000 g | Ethanol |
| 73,912 g | Wasser |
| 100,000 g | |

**Beispiel 10: Haarfestigungsschaum**

| | |
|---|---|
| 2,800 g | Chitosan |
| 0,200 g | mit 3 Mol Ethylenoxid ethoxylierter Oleylalkohol |
| 0,200 g | Cetyltrimethylammoniumchlorid |
| 0,688 g | Ameisensäure |
| 0,200 g | Parfümöl |
| 22,000 g | Ethanol |
| 73,912 g | Wasser |
| 100,000 g | |

Jeweils 100 g der Mittel nach den Beispielen 7, 8, 9 oder 10 werden mit 10 g Propan/Butan in einen für kosmetische Mittel üblichen Druckbehälter mit Schaumdüse abgefüllt. Die Haarfestigungsschäume nach den Beispielen 7, 8, 9 und 10 werden in der für derartige Mittel üblichen Art und Weise angewandt.

**Beispiel 11: Tönungsschaum**

| | |
|---|---|
| 0,5000 g | Glycerinmonolaurat |
| 0,5000 g | Cetyltrimethylammoniumchlorid |
| 0,5000 g | Distearyldimethylammoniumchlorid |
| 0,4000 g | Hydroxyethylcellulose |
| 0,0010 g | Solvent Blue 5, C.I. 42 595:1 |
| 0,0007 g | Basic Violet 14, C.I. 42 510 |
| 10,0000 g | Ethanol |
| 0,2000 g | Parfümöl |
| 87,8983 g | Wasser |
| 100,0000 g | |

100 g des Mittels nach Beispiel 11 werden mit 10 g Propan/Butan in einen für kosmetische Mittel üblichen Druckbehälter mit Schaumdüse abgefüllt.
20 g des Schaums werden im gewaschenen, handtuchtrockenen Haar verteilt. Sodann wird das Haar eingelegt und getrocknet. Die Haare sind in einem brillianten Silberton getönt und gefestigt.

**Vergleichsbeispiele**

**Beispiel A**

Im Halbseitenversuch wurden die erfindungsgemäßen Mittel gemäß den Beispielen 1, 2 und 3, die mit 2 oder 3 Mol Ethylenoxid ethoxylierten Lauryl- beziehungsweise Oleyalkohol enthalten, sowie das nicht-erfindungsgemäße Mittel gemäß Beispiel II, das mit 10 Mol Ethylenoxid ethoxylierten Oleylalkohol enthält, mit dem nicht-erfindungsgemäßen Mittel gemäß Beispiel I, das keinen ethoxylierten Fettalkohol enthält, verglichen. Die nicht-erfindungsgemäßen Mittel gemäß den Beispielen I und II wiesen die folgende Zusammensetzung auf:

Tabelle 1

| Beispiel | I | II |
|---|---|---|
| Chitosan | 0,60 g | 0,60 g |
| Oleyalkohol mit 10 Mol Ethylenoxid oxethyliert | -- | 0,50 g |
| Ameisensäure | 1,52 g | 1,52 g |
| Parfümöl | 0,75 g | 0,75 g |
| Isopropanol | 16,00 g | 16,00 g |
| Wasser | 81,13 g | 80,63 g |
| | 100,00 g | 100,00 g |

Hierzu wurde das gewaschene, handtuchtrockene mittellange Haar von 8 Versuchspersonen im Parallelversuch mit dem Mittel gemäß Beispiel I und jeweils mit einem Mittel gemäß dem Beispielen 1, 2, 3 oder II behandelt. Um eindeutige Ergebnisse zu erhalten und den Einfluß von Versuchsperson zu Versuchsperson abweichenden Haarqualitäten auszuschalten, wurde das Haar in der Mitte gescheitelt und eine Hälfte des Haares mit 8 g des Mittels gemäß Beispiel I behandelt, während die andere Hälfte des Haares jeweils mit der gleichen Menge eines der Mittel gemäß den Beispielen 1, 2, 3 oder II behandelt wurde. Sodann wurde jede Hälfte des Haares für sich durchgekämmt und hinsichtlich der Naßkämmbarkeit und der Schaumentwicklung beim Durchkämmen beurteilt. Anschließend wurde das Haar auf Wasserwellwickler gewickelt und sodann getrocknet. Nach dem Entfernen der Wickler wurde das Haar frisiert und die Frisierbarkeit und der Halt der Frisur beurteilt.

Die Ergebnisse der friseur-fachlichen Beurteilung sind in der nachfolgenden Tabelle 2 zusammengestellt. Die Bewertung erfolgte bezüglich der Naßkämmbarkeit, der Schaumentwicklung sowie der Frisierbarkeit und des Halts der Frisur nach folgendem Benotungsschema:

Benotung:

| ++ | sehr viel besser als Beispiel I |
|---|---|
| + | deutlich besser als Beispiel I |
| o | kein Unterschied zu Beispiel I |
| - | geringer als Beispiel I |

Tabelle 2

| Beispiel | II | 1 | 2 | 3 |
|---|---|---|---|---|
| Naßkämmbarkeit | o | + | ++ | ++ |
| Schaumentwicklung beim Durchkämmen | o | - | - | - |
| Frisierbarkeit und Halt der Frisur | o | o | o | o |

Die Resultate der vorstehenden Vergleichsversuche mit den erfindungsgemäßen Beispielen 1, 2, 3 und und dem nicht-erfindungsgemäßen Beispiel II zeigen, daß die erfindungsgemäßen Mittel gemäß den Beispielen 1,2 und 3 eine überraschende Verbesserung der Naßkämmbarkeit bei gleichbleibend guter Frisierbarkeit und gutem Halt der Frisur gegenüber den Mitteln gemäß den Beispielen I und II erzielt werden konnte. Zudem wurde die als negativ empfundene Schaumentwicklung beim Durchkämmen üblicher Mittel zur Festigung der Haare wie den Mitteln gemäß den Beispielen I und II bei den erfindungsgemäßen Mitteln verringert.

**Beispiel B:**

Die mechanisch-physikalische Beurteilung der Verbesserung der Naßkämmbarkeit durch die Anwendung der Mittel gemäß den erfindungsgemäßen Beispielen 4 und 5 erfolgte durch Messung der Kämmkraft in Analogie zu der in der Literatur M. L. Garcia, J. Diaz "Combabilty Measurements of Human Hair", J. Soc. Cosmet. Chem. 27 (1976), Seiten 379 bis 398 beschriebenen Methode im Vergleich zu den entsprechenden nicht-erfindungsgemäßen Beispielen III beziehungsweise IV der folgenden Zusammensetzung:

| Beispiel | III | IV |
|---|---|---|
| Polyvinylpyrrolidon | 2,00 g | 2,00 g |
| Chitosan | 0,20 g | 0,20 g |
| Parfümöle | 0,90 g | 0,90 g |
| Isopropanol | 28,00 g | 33,00 g |
| Wasser | 68,90 g | 63,90 g |
| | 100,00 g | 100,00 g |

Je eine Haarsträhne aus europäischem Humanhaar mit einem Gewicht von etwa 2 g wurde mit Wasser angefeuchtet und in das Meßgerät eingespannt. Sodann wurden jeweils 0,25 ml je eines Mittels gemäß den Beispielen 4, 5, III oder IV auf die Haarsträhne gleichmäßg aufgebracht. Anschließend wurde die zum Durchkämmen der Haarsträhne erforderliche mittlere Kämmkraft gemessen.

Die Messung der mittleren Kämmkraft erfolgte mit dem Zugprüfgerät der Firma Intron, Modell 1122 an jeweils 3 verschiedenen Haarsträhnen (Gewicht 2 g, Länge 10 cm) bei 21° Celsius unter Verwendung von 3 an einem Band rotierenden teflonbeschichteten Aluminiumkämmen (Abstand der Kämmzähne: 1 mm).

Tabelle 3 faßt die ermittelten Mittelwerte der Meßwerte der 3 Haarsträhnen zusammen.

Tabelle 3

| Beispiel | III | 4 | Kämmbarkeitsverringerung (%) |
|---|---|---|---|
| mittlere Kämmkraft (Nmm) | 5,1 | 2,8 | 45 |
| **Beispiel** | **IV** | **5** | **Kämmbarkeitsverringerung (%)** |
| mittlere Kämmkraft (Nmm) | 5,2 | 3,4 | 35 |

Wie Tabelle 3 entnommen werden kann, ist die mittlere Kämmkraft bei Verwendung der erfindungsgemäßen Mittel gemäß den Beispielen 4 und 5 deutlich geringer als bei den entsprechenden Vergleichsbeispielen III und IV.

**Beispiel C:**

Die mechanisch-physikalische Beurteilung der Verbesserung der Naß- und Trockenkämmbarkeit durch die Anwendung der erfindungsgemäßen Mittel gemäß den Beispielen 12, 13, 14 und 15 erfolgte in Analogie zu der bei Beispiel B genannten Literatur im Vergleich zu den nicht-erfindungsgemäßen Beispielen V bis X.

Je eine Haarsträhne aus europäischem Humanhaar wurde mit Wasser angefeuchtet, in das Meßgerät eingespannt und die zum Durchkämmen der Haarsträhne erforderliche maximale Kämmkraft (naß) wurde gemessen. Sodann wurden die Haarsträhnen getrocknet und erneut die zum Durchkämmen der Haarsträhne erforderliche maximale Kämmkraft (trocken) gemessen. Die Messung der maximalen Kämmkraft erfolgte mit dem Zugprüfgerät der Firma Intron, Modell 1122 an drei verschiedenen Haarsträhnen bei 21° Celsius unter Verwendung von 3 an einem Band rotierenden teflonbeschichteten Aluminiumkämmen (Abstand der Kämmzähne 1 mm).

Die Tabellen 4, 5 und 6 geben jeweils die Zusammensetzung der erfindungsgemäßen Mittel gemäß den Beispielen 12, 13, 14 und 15, der nicht-erfindungsgemäßen Vergleichsbeispiele V bis X sowie die Mittelwerte der gemessenen maximalen Kämmkräfte im nassen und getrockneten Haarzustand wieder.

Tabelle 4

| Beispiel | 12 | 13 | V | VII |
|---|---|---|---|---|
| Chitosan | 1,00 g | 1,00 g | 1,00 g | 1,00 g |
| mit 2 Mol Ethylenoxid ethoxylierter Laurylalkohol | 0,50 g | - | - | - |
| mit 3 Mol Ethylenoxid ethoxylierter Laurylalkohol | - | 0,50 g | - | - |
| mit 4 Mol Ethylenoxid ethoxylierter Laurylalkohol | - | - | 0,50 g | - |
| Ethanol | 35,00 g | 30,00 g | 20,00 g | 40,00 g |
| Pyrrolidon-Carbonsäure | 0,85 g | 0,85 g | 0,85 g | 0,85 g |
| Wasser | 62,65 g | 67,65 g | 77,65 g | 58,15 g |
| | 100,00 g | 100,00 g | 100,00 g | 100,00 g |
| maximale Kämmkraft -naß- (mN) | 236 | 255 | 306 | 354 |
| maximale Kämmkraft -trocken- (mN) | 410 | 419 | 687 | 1402 |

Tabelle 5

| Beispiel | 14 | VI | VII |
|---|---|---|---|
| Chitosan | 1,00 g | 1,00 g | 1,00 g |
| mit 2 Mol Ethylenoxid ethoxylierter Cetylalkohol | 0,50 g | - | - |
| mit 5 Mol Ethylenoxid ethoxylierter Cetylalkohol | - | 0,50 g | - |
| Ethanol | 53,00 g | 45,00 g | 40,00 g |
| Pyrrolidon-Carbonsäure | 0,85 g | 0,85 g | 0,85 g |
| Wasser | 44,65 g | 52,65 g | 58,15 g |
| | 100,00 g | 100,00 g | 100,00 g |
| maximale Kämmkraft -naß- (mN) | 239 | 290 | 354 |
| maximale Kämmkraft -trocken- (mN) | 679 | 787 | 1402 |

Tabelle 6

| Beispiel | 15 | VIII | IX | X |
|---|---|---|---|---|
| Dimethylpolysiloxan-Polyethylenoxid-Polypropylenoxid-Copolymer mit einem Trübungspunkt von 10° Celsius | 0,50 g | - | - | - |
| Dimethylpolysiloxan-Polyethylenoxid-Polypropylenoxid-Copolymer mit einem Trübungspunkt von 42° Celsius | - | 0,50 g | - | - |

Tabelle 6 (fortgesetzt)

| Beispiel | 15 | VIII | IX | X |
|---|---|---|---|---|
| Dimethylpolysiloxan-Polyethylenoxid-Polypropylenoxid-Copolymer mit einem Trübungspunkt von 80° Celsius | - | - | 0,50 g | - |
| Ethanol | 41,00 g | 40,00 g | 40,00 g | |
| Chitosan | 1,00 g | 1,00 g | 1,00 g | 1,00 g |
| Pyrrolidoncarbonsäure | 0,85 g | 0,85 g | 0,85 g | 0,85 g |
| Wasser | 56,65 g | 57,65 g | 56,65 g | 98,15 g |
| | 100,00 g | 100,00 g | 100,00 g | 100,00 g |
| maximale Kämmkraft -naß- (mN) | 212 | 250 | 313 | 272 |
| maximale Kämmkraft -trocken- (mN) | 863 | 1850 | 910 | 1509 |

Die Tabellen 4, 5 und 6 zeigen deutlich, daß die mit den erfindungsgemäßen Mitteln gemäß den Beispielen 12, 13, 14 und 15 behandelten Haarsträhnen eine deutlich verbesserte Naß- und Trockenkämmbarkeit (geringere maximale Kämmkraft) aufweisen.

**Beispiel D: Wellstabilität**

Zur Bestimmung der Wellstabilität wurde zunächst je eine Haarsträhne aus jeweils 100 Haaren auf einen Spiralwickler mit einem Durchmesser von 5 mm gewickelt und jeweils mit einem der Mittel gemäß den Beispielen 15, VIII, IX und X, deren Zusammensetzung in der Tabelle 6 angegeben ist, behandelt. Anschließend wurde die Haarsträhne getrocknet, der Wickler entfernt und die Länge der Haarsträhne (linearer Abstand zwischen den gegenüberliegenden Enden) gemessen.

Die Haarsträhne wurde anschließend 22 Stunden lang im Schrank bei 65 Prozent relativer Luftfeuchte bei 20° Celsius aufgehängt. Anschließend wurde die Länge der Haarsträhne erneut gemessen.

Aus den so erhaltenen Werten wurde der Welleffekt mit Hilfe der Gleichung

$$\text{Wellstabilität \%} = \frac{L_o - L}{L_o - L_1} \times 100$$

mit

$L_o$ = Anfangslänge der Haarsträhne
$L$ = Länge der Haarsträhne nach der Behandlung
$L_1$ = Länge der Haarsträhne im gewickelten Zustand

Die Ergebnisse dieser Vergleichsversuche sind in der folgenden Tabelle 7 zusammengefaßt.

Tabelle 7

| Beispiel | 15 | VIII | IX | X |
|---|---|---|---|---|
| Wellstabilität % | 77,8 | 76,3 | 71,6 | 61,5 |

Die Versuche zur Wellstabilität zeigen, daß die erfindungsgemäßen Mittel nicht nur die Naß- und Trockenkämmbarkeit meßbar verbessern, sondern zusätzlich auch bessere Festigungseigenschaften als die Mittel des Standes der Technik aufweisen.

**Patentansprüche**

1. Mittel zur Festigung der Haare auf der Basis einer wasserhaltigen Lösung mindestens eines fimbildenden natürlichen Polymers, dadurch gekennzeichnet, daß das filmbildende Polymer ausgewählt ist aus Chitinderivaten, Chitosan, Chitosanderivaten, Chitosansalzen, Schellack, Alginaten, Gelatine und Pektinen und daß das Mittel einen Gehalt an einer Kombination aus

(A) mindestens einem wasserlöslichen, halogenfreien organischen Lösungsmittel und
(B) 0,05 bis 2 Gewichtsprozent mindestens einer bei Raumtemperatur wasserunlöslichen, im Mittel jedoch löslichen Verbindung, welche ausgewählt ist aus $C_8$- bis $C_{20}$-Alkandiolen, $C_8$- bis $C_{20}$-Alkantriolen, den Mono- oder Monodiglyceriden der gesättigten oder ungesättigten $C_{12}$- bis $C_{20}$-Fettsäuren, den mit 2 oder 3 Mol Ethylenoxid ethoxylierten $C_{12}$- bis $C_{20}$-Fettalkoholen, Polysiloxan-Polyether-Copolymeren mit einem Trübungspunkt bei einer Temperatur unter 20° Celsius, Polyoxyethylen-Polyoxypropylen-Blockpolymeren oder Polyoxyethylen-Polyoxybutylen-Blockpolymeren,

wobei das Gewichtsverhältnis der Komponente (A) zur Komponente (B) 1000:1 bis 2,5:1 beträgt, aufweist.

2. Mittel nach Anspruch 1, dadurch gekennzeichnet, daß das wasserlösliche, halogenfreie organische Lösungsmittel der Komponente (A) ausgewählt ist aus $C_1$- bis $C_4$-Alkoholen, $C_1$- bis $C_4$-Glykolen, Polyethylenglykolestern von $C_2$- bis $C_{20}$-Carbonsäuren, flüssigen Polyethylenglykolen, Polyethylenglykolalkylethern von $C_1$- bis $C_4$-Alkoholen oder einfachen oder gemischten Ketonen von $C_1$- bis $C_5$-Alkoholen.

3. Mittel nach einem der Ansprüche 1 oder 2, dadurch gekennzeichnet, daß es 5 bis 60 Gewichtsprozent mindestens eines wasserlöslichen, halogenfreien organischen Lösungsmittels enthält.

4. Mittel nach einem der Ansprüche 1 bis 3, dadurch gekennzeichnet, daß es zur gleichzeitigen Tönung des Haares 0,01 bis 2 Gewichtsprozent eines direkt auf das Haar aufziehenden Farbstoffs enthält.

5. Mittel nach einem der Ansprüche 1 bis 4, dadurch gekennzeichnet, daß der direkt auf das Haar aufziehende Farbstoff ausgewählt ist aus aromatischen Nitrofarbstoffen, Azofarbstoffen, Anthrachinonfarbstoffen und Triphenylmethanfarbstoffen.

6. Mittel nach einem der Ansprüche 1 bis 5, dadurch gekennzeichnet, daß es 2 bis 10 Gewichtsprozent eines Treibmittels enthält, das ausgewählt ist aus n-Butan, i-Butan, Propan, Difluordichlormethan, Trichlormonofluormethan, Tetrafluordichlorethan, Dimethylether, $N_2$, $N_2O$ und $CO_2$ sowie Mischungen dieser Verbindungen.

7. Mittel nach einem der Ansprüche 1 bis 6, dadurch gekennzeichnet, daß es zusätzlich mindestens ein synthetisches, filmbildendes Polymer enthält.

8. Mittel nach einem der Ansprüche 1 bis 7, dadurch gekennzeichnet, daß es das filmbildende natürliche oder synthetische Polymer in einer Menge von 0,1 bis 5 Gewichtsprozent enthält.

## Claims

1. Hair-setting agent based on an aqueous solution of at least one film-forming natural polymer, characterized in that the film-forming polymer is selected from chitin derivatives, chitosan, chitosan derivatives, chitosan salts, shellac, alginates, gelatine and pectins and that the agent contains a combination of

(A) at least one water-soluble, halogen-free organic solvent and

(B) 0.05 to 2 per cent by weight of at least one compound which is insoluble in water but soluble in the agent at room temperature which is selected from $C_8$ to $C_{20}$-alkane diols, $C_8$ to $C_{20}$-alkane triols monoglycerides and monodiglycerides of saturated or unsaturated $C_{12}$ to $C_{20}$ fatty acids, $C_{12}$ to $C_{20}$ fatty alcohols ethoxylated with 2 or 3 moles of ethylene oxide, polysiloxane-polyether copolymers with a cloud point lower than 2o°C, polyoxyethylene-polyoxypropylene block polymers or polyoxyethylene-polyoxybutylene block polymers,

the ratio by weight of component (A) to component (B) being from 1000:1 to 2.5:1.

2. Agent according to Claim 1, characterized in that the water-soluble, halogen-free organic solvent of component (A) is selected from $C_1$ to $C_4$ alcohols, $C_1$ to $C_4$ glycols, polyethylene glycol esters of $C_2$ to $C_{20}$ carboxylic acids, liquid polyethylene glycols, polyethylene glycol alkyl ethers of $C_1$ to $C_4$ alcohols or single or mixed ketons of $C_1$ to $C_5$ alcohols.

3. Agent according to any one of Claims 1 or 2, characterized in that it includes 5 to 60 per cent by weight of at least one water-soluble, halogen-free organic solvent.

4. Agent according to any one of Claims 1 to 3, characterized in that, for simultaneous tinting of the hair, it contains 0.01 to 2 per cent by weight of a dye absorbed directly by the hair.

5. Agent according to any one of Claims 1 to 4, characterized in that the dye absorbed directly by the hair is selected from aromatic nitro dyes, azo dyes, anthraquinone dyes and triphenylmethane dyes.

6. Agent according to any one of Claims 1 to 5, characterized in that it includes 2 to 10 per cent by weight of a propellent selected from n-butane, i-butane, propane, difluorodichloromethane, trichloromonofluormethane, tetrafluorodichloroethane, dimethyl ether, $N_2$, $N_2O$ and $CO_2$, and mixtures of these compounds.

7. Agent according to any one of the Claims 1 to 6 characterized in that it additionally contains at least one synthetic film-forming polymer

8. Agent according to any one of the Claims 1 to 7, characterized in that it contains the film-forming natural or synthetic polymer in a quantity of from 0.1 to 5 per cent by weight.

**Revendications**

1. Produit de fixation des cheveux, à base d'une solution aqueuse d'au moins un polymère filmogène naturel, caractérisé en ce que le polymère filmogène est choisi parmi les dérivés de la chitine, le chitosane, les dérivés de chitosane, les sels de chitosane, la gomme laque, les alginates, la gélatine, les pectines et en ce que le produit présente une teneur d'une combinaison de

(A) au moins un solvant organique, soluble dans l'eau et exempt d'halogènes
(B) de 0,05 à 2 % en poids, d'au moins un composé insoluble dans l'eau à température ambiante, mais cependant soluble dans le produit, sélectionné parmi les alcanediols en $C_8$ à $C_{20}$, les alcanetriols en $C_8$ à $C_{20}$, les mono ou monobiglycérides des acides gras en $C_{12}$ à $C_{20}$ saturés ou insaturés, les alcools gras en $C_{12}$ à $C_{20}$ éthoxylés avec 2 ou 3 moles d'oxyde d'éthylène, les copolymères polysiloxane-polyéther avec un point de turbidité à une température inférieure à 20 °C, les polymères à blocs de polyoxyéthylène-polyoxypropyléne ou les polymères à blocs de polyoxyéthylène-polyoxybutylène ;

le rapport de poids entre les composants (A) et (B) étant de 1000 : 1 à 2,5 : 1.

2. Produit selon la revendication 1, caractérisé en ce que le solvant organique soluble dans l'eau, exempt d'halogéne, du composant (A) est choisi parmi les alcools en $C_1$ à $C_4$, les glycols en $C_1$ à $C_4$, les esters de polyéthylèneglycol d'acides carbonés en $C_2$ à $C_{20}$, les polyéthylèneglycols liquides, les polyéthylèneglycolalkyléthers d'alcools en $C_1$ à $C_4$, ou des cétones simples ou mélangées d'alcools en $C_1$ à $C_5$.

3. Produit selon l'une des revendications 1 ou 2, caractérisé en ce qu'il contient de 5 à 60 % en poids d'au moins un solvant organique soluble dans l'eau, exempt d'halogène.

4. Produit selon l'une des revendications 1 à 3, caractérisé en ce qu'il contient de 0,01 à 2 % en poids d'un colorant à appliquer directement sur les cheveux, pour réaliser simultanément une teinture des cheveux.

5. Produit selon l'une des revendications 1 à 4, caractérisé en ce que colorant à appliquer directment sur les cheveux est choisi parmi les nitrocolorants, les azocolorants, les colorants à base antrachinone et les colorants à base triphénylméthane.

6. Produit selon l'une des revendications 1 à 5, caractérisé en ce qu'il contient de 2 à 10 % en poids d'un agent soufflant, choisi parmi le n-butane, i-butane, propane, difluorodichloraméthane, trichloromonofluorométhane, tétrafluorodichloroéthane, diméthyléther, $N_2$, $N_2O$ et $CO_2$, ains que les mélanges de ces composés.

7. Produit selon l'une des revendications 1 à 6, caractérisé en ce qu'il contient en outre au moins un polymère filmogène synthétique.

8. Produit selon l'une des revendications 1 à 7, caractérisé en ce qu'il contient le polymère filmogène naturel ou synthétique en une quantité de 0,1 à 5 % en poids.